# EUROPEAN PATENT APPLICATION

(11) **EP 1 604 976 A1**
(43) Date of publication of application: **14.12.2005**
(21) Application number: 05076188.1
(22) Date of filing: 23.05.2005
(51) Int. Cl.: C07C 311/48, A61K 31/18

(54) **Nimesulide derivative method for its preparation and a pharmaceutical composition containing the same**

(30) Priority: 03.06.2004 IT MI20041121
(71) Applicant: Advance Holdings Limited, Floriana, Malta (ML)
(72) Inventor: Pifferi, Giorgio, 20147 Milano (IT)
(74) Representative: Marchi, Massimo

(57) **Abstract**

A product of Formula (I): 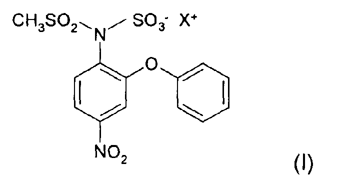 wherein X has the meanings stated in the description.

Moreover, a method of preparing the product of Formula (I) and a pharmaceutical composition that contains said product of Formula (I) are also described.

## Description

The present invention relates to a nimesulide compound of Formula (I): 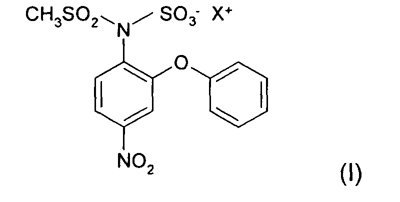 wherein
X is H or a physiologically acceptable organic or inorganic cation, a method for the preparation thereof and a pharmaceutical composition containing the same.

It is known that nimesulide (II) 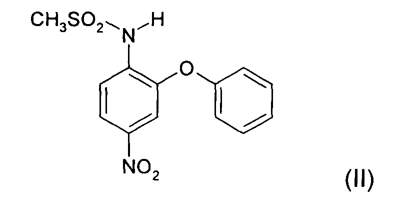 is a widely used anti-inflammatory and antipyretic agent that is useful in all inflammatory and/or painful conditions. In particular, it is often used in the treatment of acute painful conditions such as headache and toothache.

Recent studies have shown that nimesulide would inhibit preferentially COX-2 more than COX-1 cyclooxygenase (Famaey J.P., Inflamm. Res. 1997; 46: 437-446; Cullen et al. J. PET, 1998; 287: 578-582) and that would explain why nimesulide is better tolerated at the gastrointestinal level than other conventional non-steroidal anti-inflammatory drugs (NSAIDs).

A negative property of nimesulide is its poor solubility in water (approximately 0.01 mg/ml). This property has adverse consequences for bioavailability and does not allow to prepare those pharmaceutical dosage forms that imply a higher water solubility of the active principle, for example injectables, drops, syrups, mouthwashes and the like.

Since nimesulide has a hydrogen atom that is somewhat acid, it can form a salt with alkali metals or organic bases. However, the salts thus obtained generally have a low water solubility. For example, the water solubility of the sodium salt is less than 10 mg/ml. Moreover, they have the drawback of having pH values that are very high or are far from the physiological values, so that they are poorly tolerated.

The attempts reported in the literature for improving the water solubility of nimesulide include complexes with cyclodextrins (WO 91/17774 and WO 94/02177). However, the only advantage of the aqueous solutions of these complexes is that they have a pH that can be tolerated physiologically, whereas there is little increase in water solubility.

In an attempt to increase the water solubility of nimesulide, the lysine salt was also prepared (WO 95/34533), but it only reaches a solubility of 5.42 mg/ml.

An higher water solubility has been reported for the adduct with N-methyl-glucamine (WO 99/41233) and for the choline salt (EP-A-0 869 117). Indeed, according to what is reported in the above-mentioned documents, the water solubility of the adduct with N-methyl-glucamine is approximately 160 mg/ml whereas that of the choline salt is approximately 500 mg/ml.

However, the aqueous solution of the adduct with N-methyl-glucamine has the disadvantage of a high pH, so that it is poorly tolerated. In fact, as shown in example 2 of WO 99/41233, the pH value of the aqueous solution of the adduct with N-methyl-glucamine is still of 8.5-9 even after being adjusted by addition of glacial acetic acid.

In its turn, the choline salt has the drawback that it forms unstable aqueous solutions. According to EP-A-0 869 117, the stability can be increased by adding alkali carbonates, but this brings the pH to values above 8, thus compromising the tolerability of the aqueous solutions. Moreover, as is well-known, choline has a very unpleasant taste. Therefore the choline salt of nimesulide is not suitable for the preparation of oral dosage forms such as syrups and drinkable solutions.

Therefore there is still a great need for a nimesulide compound that has good solubility in water at physiological pH values and a galenically acceptable taste.

Now it has been found that the acid hydrogen atom of nimesulide can be replaced by a sulphonic group and that the product thus obtained is stable in salified form. It has further been found that the salts thus obtained are water-soluble even at the pH range between 6 and 8 so that they are well tolerated at the gastrointestinal level. Furthermore, they do not have unpleasant taste and/or odour.

However, the acid form (X = H) is rather unstable, especially in an acid medium, and undergoes hydrolysis quickly to give nimesulide and sulphuric acid. The latter, at the pH value buffered by the bodily fluids, does not cause loss of tolerability, indeed it is a physiological ionic species.

In a first aspect, therefore, the present invention relates to a product of Formula (I): 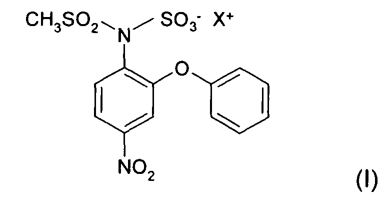 wherein
X is H or a physiologically acceptable organic or inorganic cation.

Preferably, the inorganic cation is an alkali metal or an alkaline earth metal. Typically, the inorganic cation is selected from the group comprising sodium, potassium, calcium and magnesium.

In its turn, the organic cation is preferably selected from the group comprising primary, secondary, tertiary and quaternary amines. Typically, the organic cation is selected from the group comprising mono-, di- and tri-methylamine, propylamine, 2-hydroxypropylamine, tromethamine, lysine, arginine, diethylamine, N-methylglucamine, triethanolamine, cetylpyridinium, benzalkonium, choline and the like.

The water solubility of the compounds of Formula (I) in which X is an organic or inorganic cation in the range between pH 6 and pH 8 is of from approximately 3% to 10%. For example, the water solubility of the product of Formula (I) in which X is a sodium atom is approximately 3.5 wt.% (35 mg/ml) whereas the water solubility of the product of Formula (I) in which X is choline is approximately 10 wt.%.

A second aspect of the present invention relates to a method of preparation of a product of Formula (I) in which X has the meanings stated above, comprising the following stages:
a) sulphonation of nimesulide with a sulphonating agent in the presence of a suitable organic solvent, and
b) possible salification of the product obtained in stage (a) with a suitable, physiologically acceptable organic or inorganic base.

Preferably, the sulphonating agent is chlorosulphonic acid. Advantageously, when the sulphonating agent is chlorosulphonic acid, the hydrochloric acid that forms in the course of reaction is removed from the reaction mixture by evaporation or by means of a suitable acid acceptor. In an embodiment, the acid acceptor is picoline.

Preferably, the organic solvent is selected from aprotic solvents having low polarity. Advantageously, the aforesaid solvent is a halogenated solvent. Typically, said halogenated solvent is dichloromethane.

The subsequent salification reaction is carried out by conventional methods.

A third aspect of the present invention relates to a pharmaceutical composition comprising a product of Formula (I): 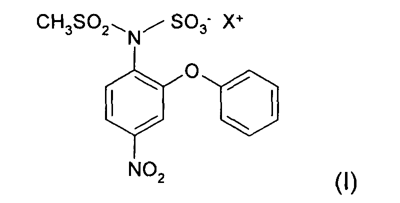 wherein
X is a physiologically acceptable organic or inorganic cation, together with a pharmaceutically acceptable vehicle.

Typical examples of pathological conditions that may benefit from treatment with a pharmaceutical composition according to the present invention are inflammatory and/or painful conditions, for example acute painful conditions such as headache and toothache.

Preferably, the pharmaceutical composition of the present invention is prepared in the form of suitable dosage forms such as, for example, capsules, coated tablets, granules, solutions and syrups for oral administration; creams, ointments, gels, mouthwashes and medicated patches for topical administration; suppositories for rectal administration and sterile solutions for intramuscular administration.

The dosage forms can also contain other traditional ingredients such as: preservatives, stabilazers, surfactants, buffers, salts for regulating the osmotic pressure, emulsifiers, sweeteners, dyes, flavourings and the like.

When required by peculiar treatments, the pharmaceutical composition of the present invention may contain other pharmacologically active ingredients whose concurrent administration may be useful.

The amount of product of Formula (I) in the pharmaceutical composition of the present invention can vary over a wide range depending on known factors such as, for example, the disorder to be treated, the severity of the disorder, the patient's body weight, the dosage form, the chosen route of administration and the number of administrations per day. However, selection of the optimum amount is simple and routine for a person skilled in the art.

Typically, the amount of product of Formula (I) in the pharmaceutical composition of the present invention will range from approximately 5 to approximately 300 mg, preferably from 20 to 150 mg. Advantageously, the daily posology will comprise the administration of an amount of product of Formula (I) such as to provide a nimesulide level of administration of from 10 to 500, preferably of from 100 to 200 mg/day.

The dosage forms of the pharmaceutical composition of the present invention can be manufactured by techniques that are well-known to a pharmaceutical chemist and comprise operations such as mixing, granulating, compressing, dissolving, sterilizing and the like.

The following examples will illustrate the present invention without limiting it in any way.

### Example 1

### (Product I, X = H)

Chlorosulphonic acid (0.79 ml; 11.83 mmol) was added dropwise to a solution of 2-picoline (2.36 ml; 23.86 mmol) in anhydrous CH₂Cl₂ (10 ml) cooled to -5°C, in such a way that the temperature was maintained at about 5°C. The resulting solution was stirred for 15 minutes, still at a temperature in the range -5°/5°C. Then a solution of nimesulide (1 g; 3.25 mmol) in CH₂Cl₂ (6 ml) was added dropwise to the aforesaid solution. The reaction mixture was left under reflux for 24 h, and after cooling to room temperature it was washed with a buffer solution at pH = 4.6 [prepared by dissolving 4.54 g of NaH₂PO₄ in 250 ml of water and adjusting the pH by adding a solution of 0.1N NaOH] and the organic phase was evaporated.

The solid residue was chromatographed on a column of silica gel (mesh 0.063-0.200), eluting with ethyl acetate.

The fractions containing the product which, in thin-layer chromatography (silica gel F₂₅₄), had Rf = 0.11 (eluent: ethyl acetate) were combined and evaporated at reduced pressure to give a solid residue consisting of Product I in which X is H.

### Example 2

### (Product I, X = Na)

The solid residue obtained in the preceding Example 1 was dissolved in the minimum amount of ethanol and treated with sodium acetate. The solution thus obtained was evaporated at reduced pressure and the residue was crystallized from isopropanol to give a crystalline product (consisting of Product I in which X is Na) having the following characteristics:
m.p. = 150-155°C (decomp.)
¹H NMR (D₂O): δ 3.49 (s, 3H); 7.20 (d, 2H, J=8.43 Hz); 7.27-7.35 (m, 1H); 7.46-7.54 (m, 2H); 7.75 (dd, 1H, J=8.79, 2.50 Hz); 7.82 (s, 1H); 8.04 (dd, 1 H, J=8.79, 2.50 Hz).
¹³C NMR (DMSO-d₆): δ 41.49 (q); 111.99 (d); 117.43 (d); 120.11 (d); 124.99 (d); 130.34 (d); 132.64 (d); 135.66 (s); 146.87 (s); 155.32 (s); 155.36 (s).
MS (ES, m/z): 316 (M⁺-PhOH); 288 (M⁺-SO₃Na-H₂O);
MS (FAB, m/z): 307 (M⁺-SO₃Na); 289 (M⁺-SO₃Na-H₂O).

### Elemental analysis for C₁₃H₁₅N₂NaO₁₀S₂; M.W. 446.37 (with 2 molecules of water of crystallization)

| | C | H | N | S |
|---|---|---|---|---|
| Calc. % | 34.98 | 3.39 | 6.98 | 14.36 |
| Found % | 35.79 | 3.00 | 5.53 | 13.95 |

Moreover, Product I in which X is Na had a solubility in water of approximately 3.5 wt.% at room temperature.

At this concentration, the aqueous solution was still stable on the seventh day at room temperature.

Acidification of the solution to about pH 2 with HCl caused decomposition of the product and precipitation of the theoretical quantity of nimesulide.

## Claims

1. A product of Formula (I): 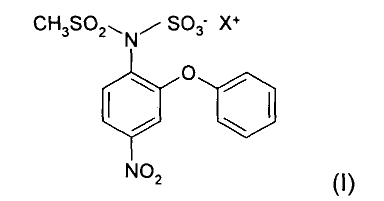 wherein
X is H or a physiologically acceptable organic or inorganic cation.

2. A product according to Claim 1, in which the inorganic cation is an alkali metal or alkaline earth metal.

3. A product according to Claim 2, in which the inorganic cation is selected from the group comprising sodium, potassium, calcium and magnesium.

4. A product according to Claim 1, in which the organic cation is selected from the group comprising primary, secondary, tertiary and quaternary amines.

5. A product according to Claim 4, in which the organic cation is selected from the group comprising mono-, di- and tri-methylamine, propylamine, 2-hydroxypropylamine, tromethamine, lysine, arginine, diethylamine, N-methylglucamine, triethanolamine, cetylpyridinium, benzalkonium and choline.

6. Method of preparation of a product of Formula (I), 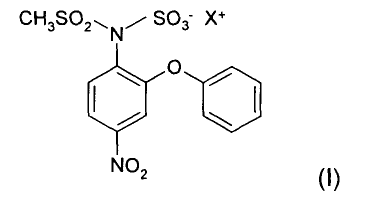 wherein
X is H or a physiologically acceptable organic or inorganic cation, comprising the following stages:
a) sulphonation of nimesulide with a sulphonating agent in the presence of a suitable organic solvent, and
b) possible salification of the product obtained in stage (a) with a suitable, physiologically acceptable organic or inorganic base.

7. A pharmaceutical composition comprising a product of Formula (I): 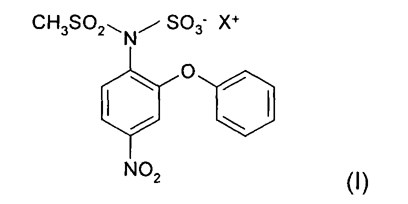 where
X is a physiologically acceptable organic or inorganic cation, together with a pharmaceutically acceptable vehicle.
